(19) Europäisches Patentamt
European Patent Office
Office européen des brevets

(11) Veröffentlichungsnummer: **0 315 197 B1**

(12) # EUROPÄISCHE PATENTSCHRIFT

(45) Veröffentlichungstag der Patentschrift: **03.02.93**

(51) Int. Cl.[5]: **A61K 31/55**, A61K 9/22

(21) Anmeldenummer: **88118429.5**

(22) Anmeldetag: **04.11.88**

(54) **Oral anzuwendende Arzneiform zur einmal täglichen Behandlung der Hypertonie mit Diltiazemhydrochlorid.**

(30) Priorität: **06.11.87 DE 3737741**

(43) Veröffentlichungstag der Anmeldung:
**10.05.89 Patentblatt 89/19**

(45) Bekanntmachung des Hinweises auf die Patenterteilung:
**03.02.93 Patentblatt 93/05**

(84) Benannte Vertragsstaaten:
**AT BE CH DE ES FR GB GR IT LI LU NL SE**

(56) Entgegenhaltungen:
**EP-A- 68 446**
**EP-A- 0 149 920**
**EP-A- 0 226 884**
**EP-A- 0 229 644**
**EP-A- 0 263 083**
**EP-A- 0 282 698**
**EP-B- 43 254**
**DE-A- 3 024 416**
**GB-A- 2 141 027**

**EUR. J. CLIN. PHARMACOL., Band 31, 1986; Seiten 423-426&NUM;**

(73) Patentinhaber: **GÖDECKE AKTIENGESELLSCHAFT**
**Salzufer 16**
**W-1000 Berlin 10(DE)**

(72) Erfinder: **Altevogt, Rudolf, Dr.**
**Am Rainhof 29**
**W-7815 Kirchzarten(DE)**
Erfinder: **Augart, Helmut**
**Tannenweg 28a**
**W-7808 Waldkirch(DE)**
Erfinder: **Bahrmann, Heinrich, Dr.**
**Am Birkenacker 7**
**W-7815 Kirchzarten-Burg(DE)**
Erfinder: **Bakovic-Alt, Renata, Dr.**
**Höchtestrasse 17c**
**W-7831 Sexau(DE)**

EP 0 315 197 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Arzneiform, die die Behandlung des Bluthochdrucks mit Diltiazem-HCl bei einmal täglicher oraler Anwendung ermöglicht.

Diltiazemhydrochlorid der Formel I

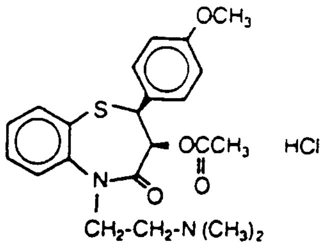

(I)

ist eine calciumantagonistisch wirkende Substanz, die vor allem zur Behandlung der Angina pectoris und der Hypertonie eingesetzt wird.

Die pharmakologischen Wirkungen beruhen im wesentlichen auf der Hemmung des zellulären Calciumeinstroms und damit einer Erniedrigung der intrazellulären Calciumkonzentration insbesondere in Zellen des Myokards und der glatten Muskulatur.

Die antihypertensive Wirkung von Diltiazem ergibt sich aus der Wirkung auf die glatte Gefäßmuskulatur. Die Hemmung des zellulären Calciumeinstroms führt hier zu einer Abnahme des Tonus der glatten Muskulatur der Gefäße; der Gefäßdurchmesser wird größer, was sich hämodynamisch in einer Verminderung des Gefäßwiderstandes der Arterien und Arteriolen und dadurch in einer Senkung des Blutdruckes äußert.

Der übliche Dosisbereich einer Therapie mit Diltiazem liegt bei 90-360 mg aufgeteilt in 3-4 Tagesdosen bei nichtretardierten Präparaten bzw. 2 Tagesdosen bei retardierten Präparaten.

Aus EP-A-68 446 ist eine retardierte Diltiazemzubereitung enthaltend 60 mg Diltiazem bekannt.

Diese Dosierschemata sind begründet durch die pharmakokinetischen Eigenschaften von Diltiazem. Aufgrund verschiedener Untersuchungen wurde bisher davon ausgegangen, daß die Wirkungen von Diltiazem, und besonders auch der gefäßerweiternde Effekt, der für die antihypertensive Wirkung entscheidend ist, parallel zu den Plasmaspiegeln verlaufen. Taeymans (Circulation 66, II-81, 1982) stellte z.B. in hämodynamischen Untersuchungen unter gleichzeitiger Bestimmung von Plasmaspiegeln fest, daß der vasodilatierende Effekt erst bei Plasmaspiegeln oberhalb von 100 ng/ml nachzuweisen war und das Ausmaß der Wirkung eng mit den Plasmaspiegeln korreliert ist. Nach Feststellungen anderer Autoren (z.B. Zelis et al. Am. J. Cardiol. 49, 529, 1982) liegen wirksame Plasmaspiegel im Bereich von ca. 50-200 ng/ml.

Der Plasmaspiegelverlauf von Diltiazem ist gekennzeichnet durch eine rasche Resorption. Nach Einnahme von unretardierten Produkten werden maximale Plasmaspiegel bereits innerhalb der ersten Stunde beobachtet. Die Halbwerzeiten für die Elimination betragen für die $\alpha$-Phase 0,1 h, für die $\beta$-Phase 2,1 h und für die $\gamma$-Phase 9,8 h, wobei die terminale Eliminationsphase ($\gamma$) im Vergleich zur $\beta$-Phase nur einen relativ kleinen Teil der Fläche unter der Plasmaspiegel-Zeit-Kurve (AUC) widerspiegelt (Kölle, Arzneim. Forsch./Drug Res. 33, 972, 1983). Daher ist für die therapeutische Wirkungsdauer hauptsächlich die $\beta$-Phase entscheidend, therapeutische Halbwertszeiten werden mit 3-5 h angegeben. Die mittleren Diltiazem-Plasmaspiegel-Verläufe nach i. v. Infusion, Einnahme einer oralen Lösung und Einnahme von Tabletten sind vergleichsweise in Abb. 1 dargestellt.

In Eur. J. Clin. Pharmacol., Band 31, 1986, S. 423-426 sind pharmakokinetische Untersuchungen mit retardierten Diltiazem-Formulierungen beschrieben bei 2 mal täglichen Gaben von 120 mg bzw. 180 mg.

Aufgrund dieser pharmakokinetischen und pharmakodynamischen Substanzeigenschaften wurden bisher Tabletten entwickelt, die zur 3-4mal täglichen Einnahme vorgesehen sind (z.B. 3 mal 1 Tablette zu 60 mg), bzw. die zur zweimal täglichen Einnahme (z.B. 2 mal 1 Tablette zu 90 mg) vorgesehen sind.

Mit diesen Arzneiformen lassen sich bei den oben genannten Einnahmeschemen im steady state Plasmaspiegel erzielen, die über 24 h weitgehend innerhalb des als wirksam angesehenen Bereiches liegen.

So betrugen nach Gabe von dreimal täglich 1 Tablette zu 60 mg die mittleren Plasmaspiegel (css) 63,7 ng/ml, nur kurzfristig (nachts) wurde ein Wert von 50 ng/ml unterschritten. Nach Gabe von zweimal täglich 90 mg in Form von Retardtabletten wurden mittlere Plasmaspiegel von 67,2 ng/ml und Plasmaspiegelminima von 55,8 ng/ml gemessen. Zur Verdeutlichung sind die so erzielten Plasmaspiegel-Verläufe in Abb. 2 dargestellt.

Zur Behandlung der Hypertonie jedoch ist aus Compliance-Gründen eine Arzneiform, die nur einmal täglich einzunehmen ist, sehr erwünscht.

Aufgabe der Erfindung ist es daher, eine Arzneiform zur Therapie des Bluthochdrucks zur Verfügung zu stellen, die bei einmal täglicher Gabe über 24 Stunden zu einer konstanten Senkung des Blutdrucks auf Normalwerte führt und dabei trotzdem weder zu überhöhten Plasmaspiegeln in der Anflutphase, noch zur Dosiserhöhung in den Unverträglichkeitsbereich, noch zu Wirkstoffverlusten während der Darmpassage führt.

Die Entwicklung einer retardierten oralen Arzneiform, die bei nur einmal täglicher Gabe über 24 h therapeutisch ausreichende Plasmaspiegel (50-200 ng/ml) liefert, erscheint jedoch aus folgenden Gründen mit den üblichen Retardverfahren nicht möglich:
Die kurze Halbwertszeit der Wirksubstanz Diltiazem von 3-5 h würde eine extreme Retardierung des Produktes (z.B. vollständige Freisetzung des Wirkstoffes erst nach ca. 17-19 h) notwendig machen. Da aber die Darmpassage beim Menschen durchschnittlich lediglich 8 h dauert, ist anzunehmen, daß bei einer derart ausgeprägten Retardierung eine Großteil des Wirkstoffes nicht mehr resorbiert werden kann, weil er sich bereits nach ca. 8 h entweder im unteren Darmabschnitt (Colon bzw. Rektum) befindet, aus dem bekanntermaßen kaum noch eine Resorption erfolgt, oder zu diesem Zeitpunkt bereits mit den Feces ausgeschieden ist und daher überhaupt nicht mehr zur Resorption zur Verfügung steht. Die Folge ist in beiden Fällen ein Bioverfügbarkeitsverlust, der individuell je nach Dauer der Darmpassage unterschiedlich stark ausgeprägt ist.

Die vorliegende Erfindung bezieht sich auf eine retardierte orale Darreichungsform von Diltiazemhydrochlorid, mit der ohne die o.g. Bioverfügbarkeitsverluste eine zuverlässige Therapie der Hypertonie bei einmal täglicher Gabe möglich ist. Überraschenderweise wurde nämlich gefunden, daß eine 24 Stunden anhaltende klinisch ausreichende Blutdrucksenkung mit einer oral einzunehmenden Arzneiform möglich ist, die nur in einem solchen Maße retardiert ist, daß gegenüber den zu zwei- bis dreimal täglicher Gabe vorgesehenen Arzneimitteln kein Bioverfügbarkeitsverlust eintritt. Nach Einnahme einer solchen Arzneiform werden Plasmaspiegel im bisher als wirksam angesehenen Bereich von im Mittel 50-200 ng/ml nur jeweils über ca. 16 h nach Einnahme der Tabletten erreicht. Dennoch ist wider Erwarten die Blutdrucksenkung nach 24 h nach der letzten Einnahme einer Tablette noch so ausgeprägt wie 12 h nach Einnahme einer konventionellen Retardtablette, die zweimal täglich einzunehmen ist.

Gegenstand der Erfindung sind daher Arzneimittel enthaltend Diltiazemhydrochlorid in Dosierungen von 90-270 mg, bevorzugt 120-240 mg und ganz besonders bevorzugt 180 mg, bei einer in-vitro-Freisetzungsrate des Wirkstoffes von 20 - 70 %, vorzugsweise 35-50 % nach 1 h, 35 - 85 %, vorzugsweise 50-70 % nach 2 h, 50 - 100%, vorzugsweise 70-95 % nach 3,5 h und nach 5 h mindestens 60 %, vorzugsweise mindestens 85 % zur Behandlung der Hypertonie durch eine einmal tägliche orale Gabe sowie deren Herstellung und deren Verwendung.

Die erfindungsgemäße Arzneiform kann zur oralen Applikation in den üblichen galenischen Formulierungen wie zum Beispiel Retardtabletten, Retardtabletten in Kapseln, Pelletts in Kapseln vorliegen, und neben dem Wirkstoff Diltiazemhydrochlorid die üblichen pharmakologisch unbedenklichen Träger- und Zusatzstoffe enthalten.

Derartige Zusätze sind z. B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyäthylenglykol). Diese für orale Applikationen geeigneten Zubereitungsformen können gewünschtenfalls zusätzliche Geschmacks- und/oder Süßstoffe enthalten.

Im folgenden soll die Erfindung anhand von Ausführungsbeispielen erläutert werden:

Beispiel 1

45,0 kg Diltiazemhydrochlorid, 127,5 kg Lactose, 18,75 kg hydriertes Rizinusöl und 30,0 kg Stearinsäure werden unter ständigem Rühren auf 60 ° C erhitzt. Die erhaltene Mischung wird langsam auf Raumtemperatur abgekühlt und anschließend über ein 1 mm Sieb gegeben. Danach werden 2,25 kg Natriumcarboxymethylcellulose und 0,75 kg Magnesiumstearat hinzugemischt. Das erhaltene Granulat wird zu Tabletten verpresst. Der Wirkstoff wurde aus diesen Tabletten wie folgt freigesetzt (Prüfung in vitro nach National Formulary XIV):

Nach 1 h: 38,9 %
2 h: 56,1 %
3,5 h: 75,6 %
5 h: 88,4 %

Die Plasmaspiegelverläufe nach Einzelgabe von jeweils 180 mg Diltiazemhydrochlorid in der oben beschriebenen Tabletten-Formulierung wurden an 6 gesunden Probanden mittels einer empfindlichen und spezifischen GC-Methode ermittelt.

Es ergaben sich folgende Parameter:

Cmax: 123,8 ± 42,5 ng/ml
Tmax: 4,2 ± 1,0 h
AUC: 1658 ± 678 mg x h/ml

Im steady state ergibt sich unter Verwendung der bekannten pharmakokinetischen Mikrokonstanten von Diltiazem der in Abb. 3 dargestellte Plasmaspiegelverlauf.

Beispiel 2

5,092 Kg Lactose, 7,2 Kg Diltiazemhydrochlorid, 2,12 kg feinpulverisiertes hydriertes Rizinusöl und 2,996 kg Stearinsäure DAC Pulver werden gemischt und unter ständigem Rühren auf 60 ° C erhitzt. Die erhaltene Mischung läßt man abkühlen und verarbeitet sie zu einem feinen Granulat. Dem Granulat werden 0,34 kg Methylcellulose MH 300 pulvis und 0,092 kg Magnesiumstearat zugefügt. Auf einer Tablettenpresse werden mit einem Preßdruch von 0,6 bis 0,8 Tonnen Tabletten mit einem Gewicht von 446 mg gepreßt.

Beispiel 3

5,72 kg Lactose, 7,2 kg Diltiazemhydrochlorid, 1,92 kg feinpulverisiertes hydriertes Rizinusöl, 2,728 kg Stearinsäure DAC Pulver und 0,09 kg Hydroxyethylcellulose/Natrosol 250 NHX werden gemischt und unter ständigem Rühren auf 60 ° C erhitzt. Die erhaltene Mischung läßt man abkühlen und verarbeitet sie zu einem feinen Granulat. Dem Granulat werden 0,092 kg Magnesiumstearat zugefügt. Auf einer Tablettenpresse werden mit einem Preßdruch von 0,6 bis 0,8 Tonnen Tabletten mit einem Gewicht von 443,75 mg gepreßt.

Es zeigt sich, daß ein bisher als therapeutisch wirksam angesehener Plasmaspiegel von mindestens 50 ng/ml nur über ca. 16 h aufrecht erhalten wird und somit im steady state für ca. 8 h unterschritten wird. 24 h nach Einnahme der Tabletten liegen die Spiegel nach Einmalgabe bei ca. 20 ng/ml, im steady state bei ca. 25 ng/ml.

Für den Fachmann völlig überraschend zeigte sich in klinischen Prüfungen jedoch, daß trotz dieses 8-stündigen deutlichen Unterschreitens der bisher als minimal wirksam angesehenen Plasmaspiegel mit 180 mg Diltiazemhydrochlorid in der genannten Arzneiform eine fortdauernde antihypertensive Wirkung erzielt werden kann, die der der bisherigen Anwendung mit Plasmaspiegeln entsprechend dem bisherigen Stand der Erkenntnisse (z.B. 2 x täglich 90 mg Diltiazem in Retardform) mindestens gleichzusetzen ist:

In einer multizentrischen, randomisierten Doppelblind-Parallel-Studie wurde bei Patienten mit Hypertonie der WHO-Klassifizierung I und II sowie III (bei gleichzeitiger koronarer Herzkrankheit) die blutdrucksenkende Wirkung von einmal täglich 180 mg mit der von zweimal täglich 90 mg Diltiazem verglichen. Die Patienten befanden sich stationär in Reha-Kliniken.

Nach Absetzen einer eventuellen antihypertensiven Vorbehandlung erhielten die Patienten zunächst 1 Woche Placebo. Daran schloß sich eine dreiwöchige Behandlung mit entweder morgens 180 mg und abends Placebo oder morgens und abends je 90 mg Diltiazem an. Von 104 Patienten verblieben nach Abzug von Placeborespondern für die Wirksamkeitsanalyse 82 Patienten (40 Patienten in der 1 x 180 mg-Gruppe und 42 in der 2 x 90 mg-Gruppe).

Zur Beurteilung der Wirksamkeit wurde der diastolische Blutdruck im Sitzen zu 3 verschiedenen

Tageszeiten (8, 11 und 17 Uhr) herangezogen. "Response" war definiert als eine Senkung des diastolischen Blutdrucks um mindestens 10 mmHg oder unter einen Wert von 90 mmHg.

Nach dreiwöchiger Behandlung war in beiden Behandlungsgruppen der diastolische Blutdruck im Vergleich zum Ende der Placebophase deutlich gesenkt. Die Abnahme betrug in beiden Gruppen 13-16 mmHg (in Abhängigkeit vom Meßzeitpunkt) ohne singifikanten Unterschied zwischen den Gruppen. Dieses gilt insbesondere auch für die Messung um 8 Uhr, die vor der Medikation erfolgte ("pre-dose"), also Aufschluß über die 24-h-Wirkung von 1 x 180 mg Diltiazem gibt. Diese Ergebnisse werden durch die grafische Darstellung in Abb. 4 verdeutlicht.

Die globale Response-Rate (Anteil der Patienten mit Response zu allen drei Meßzeitpunkten) betrug 85 % in der 1 x 180 mg-Gruppe und 62 % in der 2 x 90 mg-Gruppe. Die Response-Rate zum Zeitpunkt 8 Uhr, die wiederum die 24-h-Wirkung von 3 x täglich 180 mg widerspiegelt, betrug 87,5 % in der 1 x 180 mg-Gruppe und 81 % in der 2 x 90 mg-Gruppe. Die Ergebnisse sind in der folgenden Tabelle 1 dargestellt.

Tabelle 1

| Variable | 1 × 180 mg (n = 40) Basislinie | Endpunkt | 2 × 90 mg (n = 42) Basislinie | Endpunkt |
|---|---|---|---|---|
| DBD sitzend | | | | |
| 8 a.m. | 98.3 (3.9) | 85.3 (8.3) | 99.8 (5.2) | 83.9 (10.7) |
| 11 a.m. | 98.5 (3.9) | 83.3 (8.2) | 100.2 (5.4) | 85.4 (9.5) |
| 5 p.m. | 99.2 (3.9) | 85.2 (8.8) | 99.9 (5.9) | 87.0 (10.7) |
| DBD stehend | | | | |
| 8 a.m. | 100.1 (7.1) | 87.3 (9.8) | 102.0 (5.8) | 88.5 (9.7) |
| 11 a.m. | 100.5 (5.8) | 87.1 (8.3) | 102.9 (5.6) | 87.3 (10.6) |
| 5 p.m. | 101.0 (4.8) | 88.8 (8.9) | 102.5 (5.8) | 87.7 (9.2) |
| SBD sitzend | | | | |
| 8 a.m. | 161.3 (16.2) | 141.3 (14.2) | 156.8 (17.2) | 141.1 (14.9) |
| 11 a.m. | 159.2 (18.2) | 137.6 (12.2) | 158.1 (16.5) | 140.1 (15.8) |
| 5 p.m. | 158.9 (16.1) | 140.2 (14.2) | 156.4 (15.6) | 141.1 (15.6) |
| SBD stehend | | | | |
| 8 a.m. | 158.6 (16.4) | 141.0 (14.8) | 155.5 (17.9) | 137.4 (16.1) |
| 11 a.m. | 157.9 (19.6) | 137.1 (14.2) | 155.8 (17.7) | 135.7 (15.5) |
| 5 p.m. | 158.4 (16.3) | 138.5 (14.8) | 156.7 (15.0) | 138.6 (15.1) |
| HR sitzend | | | | |
| 8 a.m. | 76.8 (9.4) | 73.7 (7.1) | 76.4 (9.6) | 72.1 (8.2) |
| 11 a.m. | 75.1 (9.2) | 71.1 (7.0) | 76.0 (9.1) | 71.3 (7.8) |
| 5 p.m. | 76.5 (9.2) | 70.0 (7.2) | 77.0 (8.4) | 72.1 (8.1) |
| HR stehend | | | | |
| 8 a.m. | 87.2 (11.0) | 80.6 (7.4) | 85.1 (9.1) | 79.0 (8.0) |
| 11 a.m. | 84.4 (9.5) | 78.8 (6.9) | 83.6 (10.4) | 78.6 (8.6) |
| 5 p.m. | 85.6 (10.3) | 78.6 (7.7) | 85.8 (9.1) | 79.6 (9.1) |
| Körpergewicht (kg) | 82.5 (13.8) | 79.7 (12.6) | 83.7 (15.7) | 80.9 (14.2) |

DBD = Dialostischer Blutdruck (mmHg)
SBP = Systolischer Blutdruck (mmHg)
HR = Puls (1/min)

Die Standardabweichung ist in Klammern angegeben

Der systolische Blutdruck im Sitzen wurde in der 1 x 180 mg-Gruppe um 19-22 mmHg und in der 2 x 90 mg-Gruppe um 15-18 mmHg gesenkt.

Die Senkung des systolischen Drucks im Stehen lag in der gleichen Größenordnung. In beiden Behandlungsgruppen wurde ohne erkennbaren Unterschied zwischen den Gruppe die unter Diltiazem bekannte, leichte Senkung der Herzfrequenz (um 4-8 Schläge pro Minute) beobachtet.

Bezüglich der antihypertensiven Wirkung ergab sich somit zwischen den Behandlungsgruppen kein Unterschied. Überraschenderweise ergaben sich auch keine Unterschiede bezüglich der Art, Schwere und Häufigkeit von Nebenwirkungen, obwohl nach einmal täglicher Gabe von 180 mg Diltiazem deutlich höhere Plasmaspiegelmaxima als nach zweimal täglich Gabe von 90 mg auftreten. Zur Veranschaulichung ist dieser überraschende Tatbestand in Abb. 6 dargestellt. Bei der Darreichung von 2 mal täglich 90 mg Diltiazem in einer Retardformulierung wird der in der Kurve dargestellte Plasmaspiegelverlauf erhalten.

Erwartungsgemäß wird für die gleiche Formulierung mit 180 mg Diltiazem, die einmal täglich verabreicht wird, die gleichfalls dargestellte Kurve mit wesentlich höherer Anflutung innerhalb der esten sechs Stunden und einem erheblich stärkeren Absinken des Plasmaspiegels bis zum Ende der 24-Stundenperiode erreicht. Eine derartige Anflutung des Medikaments und das starke Absinken gegen Ende der Ennahmeperiode ist normalerweise äußerst unerwünscht. Angestrebt werden möglichst konstante Plasmaspiegel über den gesamten Therapiezeitraum und die Herstellung einer Retardformulierung gemäß der vorliegenden Erfindung war daher nicht naheliegend. Umso überraschender ist es daher, daß die therapeutische Wirkung der erfindungsgemäßen Formulierung zu einer fast konstanten Blutdrucksenkung über den gesamten Verabreichungszeitraum führt, wie es im Balkendiagramm der Abb. 6 dargestellt ist.

Die im stationären Bereich gemachten Erfahrungen wurden in einer weiteren Studie an 87 ambulanten Patienten mit Hypertonie bestätigt. In diese multizentrische randomisierte Doppelblind-Parallel-Studie wurden wiederum Hypertoniker der WHO-Klasse I-III eingeschlossen, deren diastolischer Blutdruck am Ende der Placebophase höher als 95 mmHg war. Einer 2-wöchigen Placebophase folgte eine Behandlungsphase von 8 Wochen. Das Therapieschema von 1 x tgl. 180 mg bzw. 2 x tgl. 90 mg glich dem der vorangegangenen Studie. Zur Beurteilung der Wirksamkeit wurde die Senkung des diastolischen Blutdrucks bei der Messung am Morgen herangezogen, die jeweils nach 8 Wochen Behandlung erzielt worden war. Die Blutdruckmessung erfolgte wiederum vor der Medikation, um Aufschluß über die 24-h-Wirkung bei der einmal täglichen Behandlung zu erhalten.

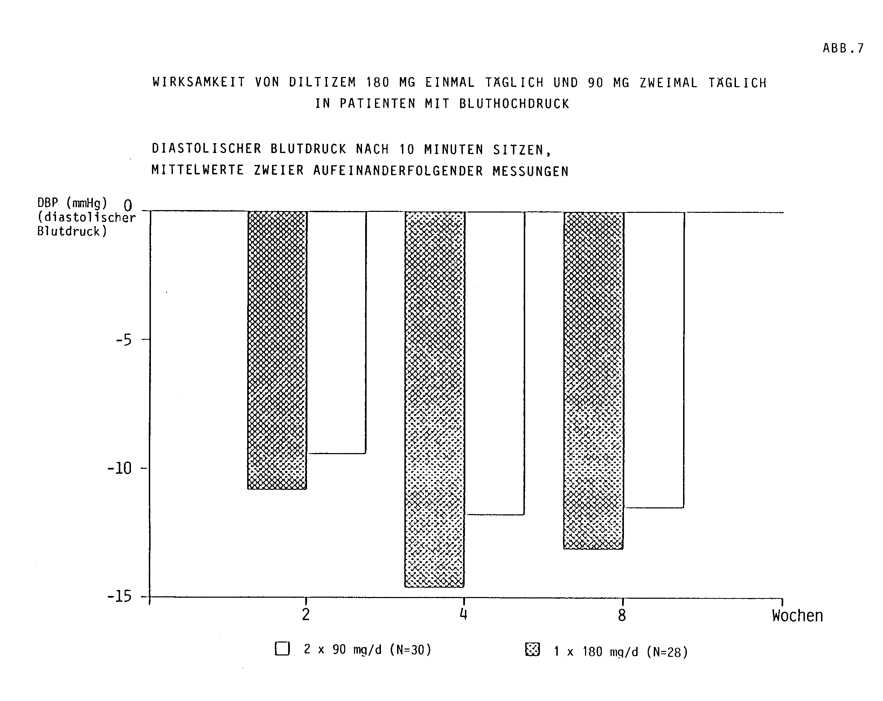

Nach 8-wöchiger Behandlung war der diastolische Blutdruck im Sitzen im Mittel gegenüber der Placebophase in der 1 x 180 mg-Gruppe um 13 mmHg und in der 2 x 90 mg-Gruppe um 12 mmHg gesenkt. Ein statistisch signifikanter Unterschied zwischen beiden Gruppen war nicht festzustellen. Diese Ergebnisse sind in Abb. 5 und Abb. 7 grafisch dargestellt.

Die Response-Rate (definiert wie in der zuvor diskutierten Prüfung) betrug in der 1 x 180 mg-Gruppe 80 % und in der 2 x 90 mg-Gruppe 70 %.

Der mittlere systolische Blutdruck im Sitzen war um 16 mmHg (1 x 180 mg) bzw. 10 mmHg (2 x 90 mg) gesenkt. Eine ähnliche Abnahme des systolischen Blutdrucks ergab sich auch nach 3 Minuten Stehen. In beiden Behandlungsgruppen ergaben sich wieder leichte Abnahmen der Herzfrequenz ohne Unterschied zwischen den Gruppen. Bezüglich der Nebenwirkungen wurden ebenfalls keine Unterschiede zwischen den Behandlungsgruppen festgestellt. Die Ergebnisse der Versuche sind in der folgenden Tabelle 2 dargestellt:

**Tabelle 2**

| Variable | 1 x 180 mg (n = 30) Basislinie | Endpunkt | 2 x 90 mg (n = 30) Basislinie | Endpunkt |
|---|---|---|---|---|
| DBD sitzend | 100.7 (3.0) | 87.8 (9.1) | 99.1 (3.2) | 88.4 (8.0) |
| DBD stehend | 98.7 (3.2) | 84.8 (9.2) | 99.0 (3.1) | 86.9 (8.6) |
| SBD sitzend | 175.1 (16.6) | 159.0 (18.8) | 169.4 (19.7) | 159.2 (23.7) |
| SBD stehend | 169.7 (19.8) | 150.5 (26.6) | 168.5 (22.1) | 156.7 (28.4) |
| HR sitzend | 72.5 (4.9) | 68.7 (4.4) | 73.5 (6.5) | 69.8 (6.0) |
| HR stehend | 74.9 (6.3) | 71.3 (5.2) | 75.9 (7.5) | 72.5 (7.0) |
| Körpergewicht (kg) | 80.3 (13.6) | 79.9 (13.5) | 79.2 (12.9) | 79.0 (12.9) |

DBD = Dialostischer Blutdruck (mmHg)

SBP = Systolischer Blutdruck (mmHg)

HR = Puls (1/min)

Die Standardabweichung ist in Klammern angegeben

Auch bei dieser Studie unter praxisnahen Bedingungen an ambulanten Patienten ergaben sich bezüglich der antihypertensiven Wirksamkeit keine statistisch signifikanten Unterschiede zwischen beiden Behandlungsgruppen. Tendenziell deutet sich hier sogar ein Vorteil bei der einmal täglichen Gabe von 180 mg Diltiazem an. Aufgrund der pre-dose-Messungen konnte der antihypertensive Effekt von Diltiazem bei einmal täglicher Einnahme der beschriebenen Arzneiform über 24 Stunden nachgewiesen werden.

**Patentansprüche**

1. Retardierte Arzneimittel für eine einmal tägliche orale Gabe enthaltend Diltiazemhydrochlorid in Dosierungen von 90-270 mg bei einer in-vitro-Freisetzungsrate nach National Formulary XIV des Wirkstoffes von 20 - 70% nach 1 h, 35 - 85% nach 2 h, 50 - 100% nach 3,5 h und mindestens 60% nach 5 h , wobei Formulierungen, bei denen die Freisetzung des Wirkstoffes durch eine Membrane erfolgt, ausgenommen sind.

2. Retardierte Arzneimittel gemäß Anspruch 1 enthaltend Diltiazemhydrochlorid in Dosierungen von 180 mg bei einer in-vitro-Freisetzungsrate des Wirkstoffes von 35 - 50% nach 1 h, 50 - 70% nach 2 h , 70 - 95% nach 3,5 h und mindestens 85% nach 5 h.

3. Verfahren zur Herstellung von Arzneimitteln gemäß Anspruch 1, dadurch gekennzeichnet, daß man Diltiazemhydrochlorid in einen pharmakologisch unbedenklichen Trägerstoff unter Retardierung so einarbeitet, daß das fertige Arzneimittel eine in-vitro-Freisetzungsrate nach National Formulary XIV aufweist, wonach nach 1 h 20 - 70%, nach 2 h 35 - 85%, nach 3,5 h 50 - 100% und nach 5 h mindestens 60% freigegeben werden.

4. Verfahren nach Anspruch 3, dadurch gekennzeichnet, daß das fertige Arzneimittel eine in-vitro-Freisetzungsrate nach National Formulary XIV aufweist, wonach nach 1 h 35 - 50%, nach 2 h 50 - 70%, nach 3,5 h 70 - 95% und nach 5 h mindestens 85% freigegeben sind.

5. Verwendung von Diltiazemhydrochlorid zur Herstellung von retardierten Arzneimitteln gemäß der Ansprüche 1 und 2 für eine einmal tägliche orale Gabe bei der Behandlung der Hypertonie.

**Claims**

1. A slow-release drug for a single daily oral administration containing diltiazem hydrochloride in dosages of 90 to 270 mg with an in vitro liberation rate according to National Formulary XIV of the active material of 20 to 70% after 1 hour, of 35 to 85% after 2 hours, of 50 to 100% after 3.5 hours and of at least 60% after 5 hours, excluding formulations in which the liberation of the active material occurs through a membrane.

2. A slow-release drug according to claim 1 containing diltiazem hydrochloride in dosages of 180 mg at an in vitro liberation rate of the active material of 35 to 50% after 1 hour, of 50 to 70% after 2 hours, of 70 to 95% after 3.5 hours and of at least 85% after 5 hours.

3. A process for the preparation of drugs according to claim 1, characterised in that diltiazem hydrochloride is incorporated into a pharmacologically acceptable carrier material with retardation so that the finished drug has an in vitro liberation rate according to National Formulary XIV, according to which liberation occurs from 20-70% after 1 hour, 35-85% after 2 hours, 50-100% after 3.5 hours and at least 60% after 5 hours.

4. A process according to claim 3, characterised in that the finished drug has an in vitro liberation rate according to National Formulary XIV according to which liberation occurs from 35-50% after 1 hour, 50-70% after 2 hours, 70-95% after 3.5 hours and at least 85% after 5 hours.

5. Use of diltiazem hydrochloride for the preparation of slow-release drugs according to claims 1 and 2 for the treatment of hypertension by a single daily oral administration.

**Revendications**

1. Médicament à effet retard pour une prise orale journalière unique contenant de 90 à 270 mg de chlorhydrate de diltiazem, avec une cinétique de libération in vitro, telle que définie par le National Formulary XIV, du principe actif de 20 à 70% après 1 heure, 35 à 85% après 2 heures, 50 à 100% après 3,5 heures et au moins 60% après 5 heures, les formulations étant ainsi assimilées après libération du principe actif au travers d'une membrane.

2. Médicament à effet retard selon la revendication 1, contenant 180 mg de chlorhydrate de diltiazem, avec une cinétique de libération in vitro du principe actif de 35 à 50% après 1 heure, de 50 à 70% après 2 heures, de 70 à 95% après 3,5 heures et d'au moins 85% après 5 heures.

3. Procédé de préparation d'un médicament selon la revendication 1, caractérisé en ce que l'on mélange du chlorhydrate de diltiazem avec un support pharmacologiquement acceptable à effet retard, de sorte que le médicament final montre une cinétique de libération, telle que définie par le National Formulary XIV, de 20 à 70% après 1 heure, 35 à 85% après 2 heures, 50 à 100% après 3,5 heures et au moins 60% après 5 heures.

4. Procédé selon la revendication 3, caractérisé en ce que le médicament final montre une cinétique de libération, telle que définie par le National Formulary XIV, de 25 à 50% après 1 heure, 50 à 70% après 2 heures, 70 à 95% après 3,5 heures et au moins 85% après 5 heures.

5. Utilisation de chlorhydrate de diltiazem pour la préparation d'un médicament à effet retard selon la revendication 1 ou 2, pour une prise orale journalière unique pour le traitement de l'hypertension.

ABB. 1

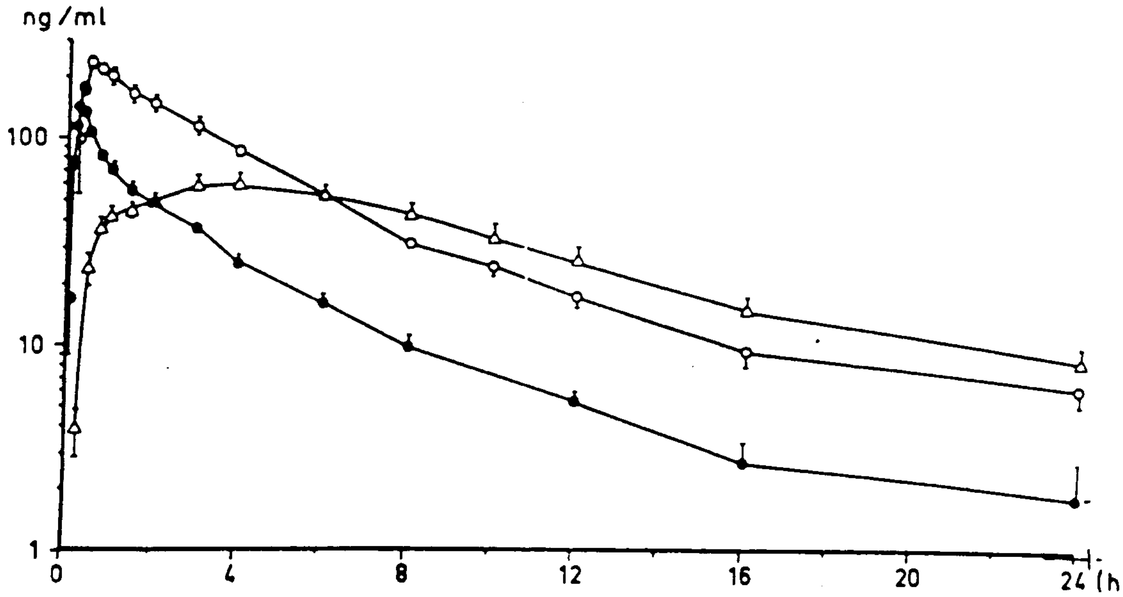


Mittlerer Diltiazem Plasmaspiegel nach i.v. Infusion von 20 mg (●), einer peroralen Lösung von 120 mg (○) und zwei 60 mg-Tabletten (△) an 6 Probanden.

ABB.2

Mittlerer Diltiazem-Plasmaspiegel nach i.v.-Infusion von 20 mg (●), einer peroralen Lösung von 120 mg (○) und zwei 60 mg Tabletten (Δ) an 6 Probanden

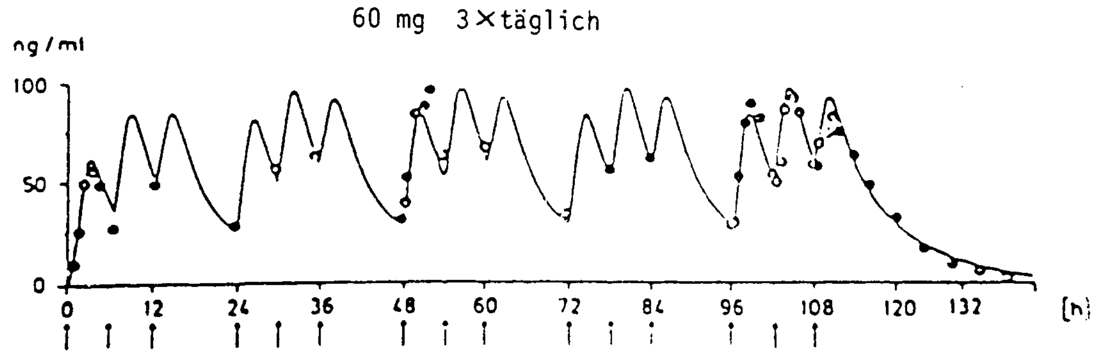


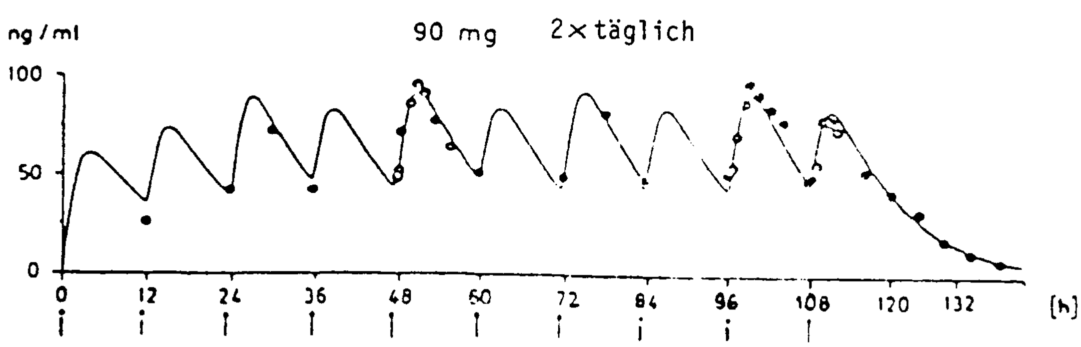


Diltiazem Plasma-Spiegel bei fünfmaliger Gabe von 180 mg täglich (Mittelwerte von 12 Probanden)

ABB. 3

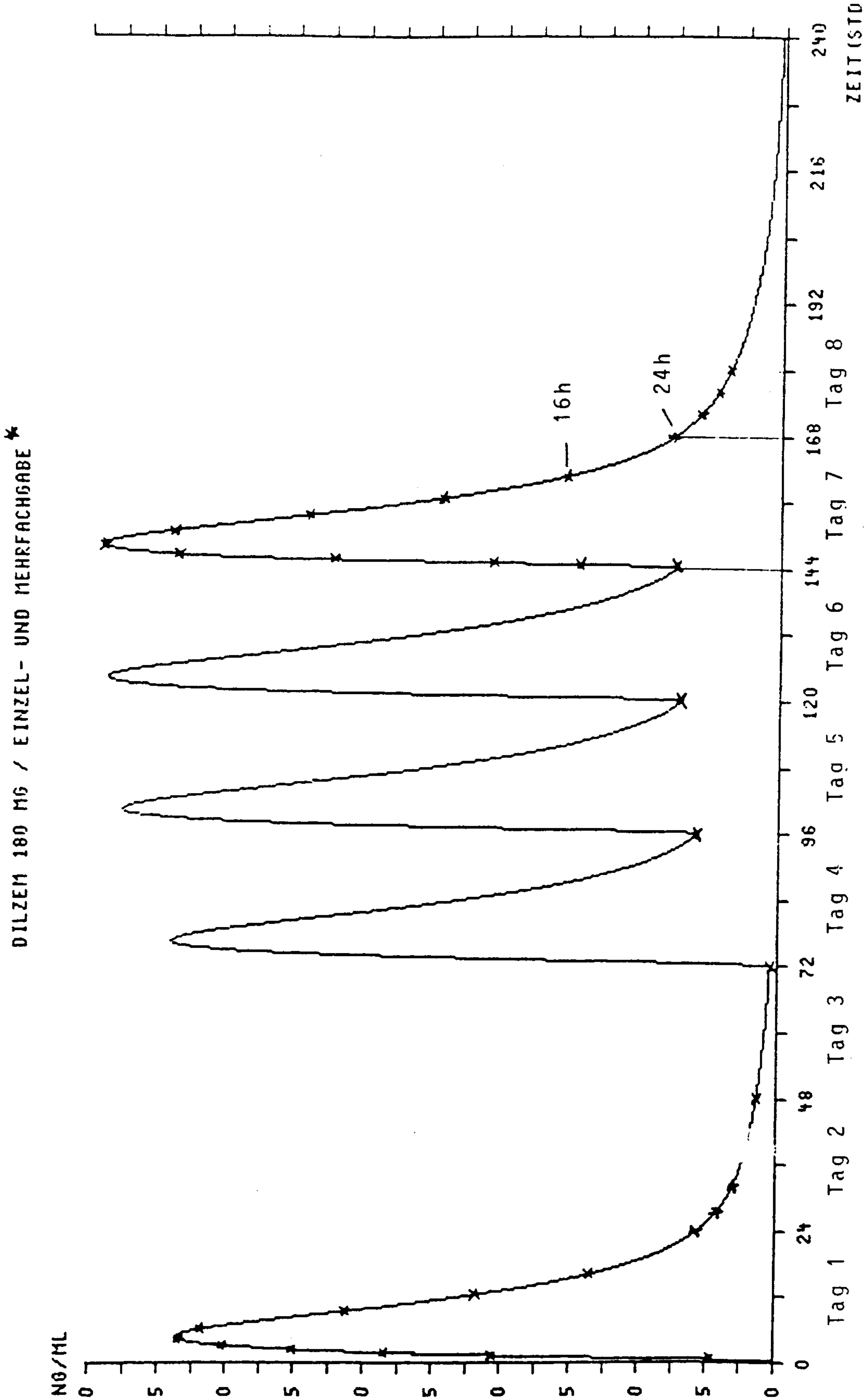
DILZEM 180 MG / EINZEL- UND MEHRFACHGABE *
NG/ML
150
135
120
105
90
75
60
45
30
15
0
0
24
48
72
96
120
144
168
192
216
240
ZEIT (STD.)
Tag 1
Tag 2
Tag 3
Tag 4
Tag 5
Tag 6
Tag 7
Tag 8
16h
24h

ABB. 4

WIRKUNG VON DILTIAZEM 180 MG EINMAL TÄGLICH UND 90 MG ZWEIMAL TÄGLICH
BEI PATIENTEN MIT BLUTHOCHDRUCK

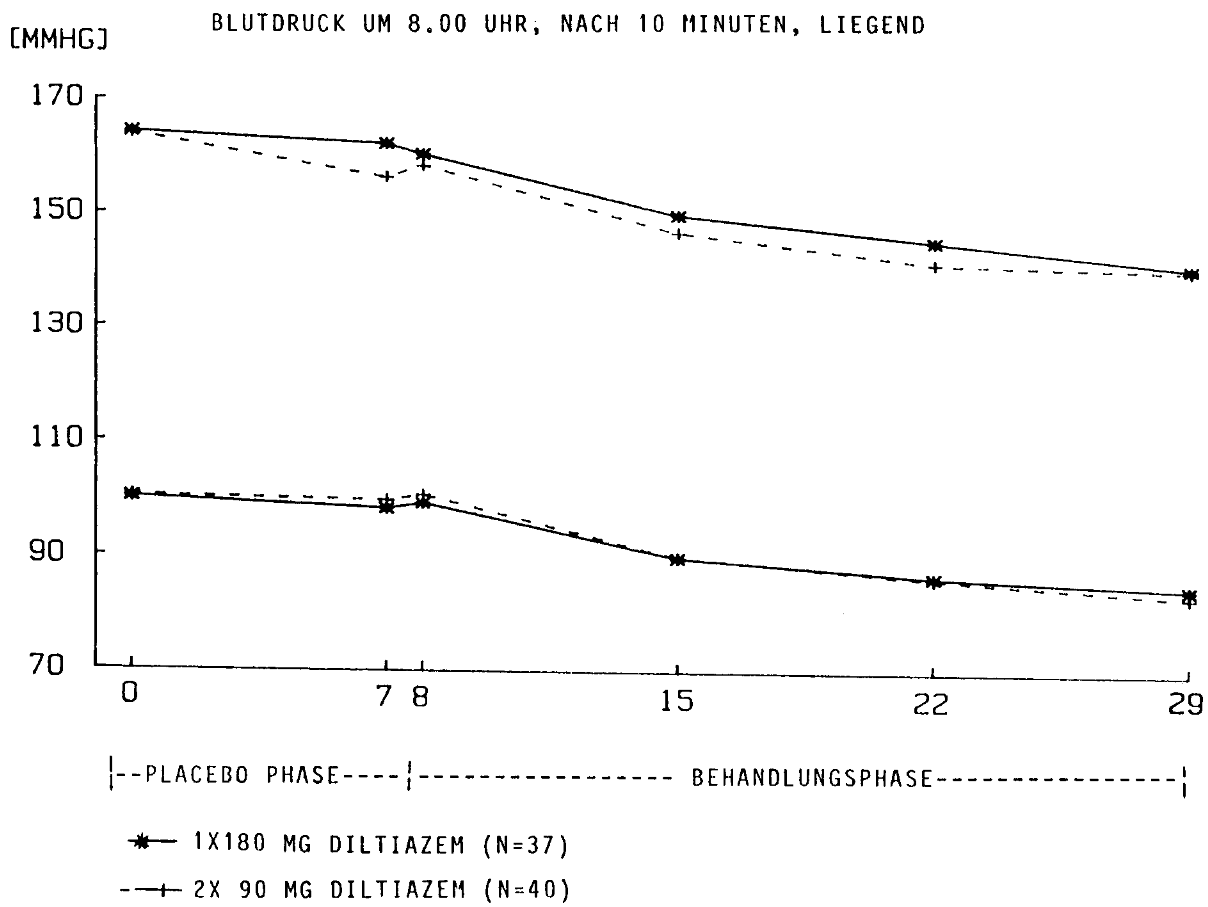

ABB.5

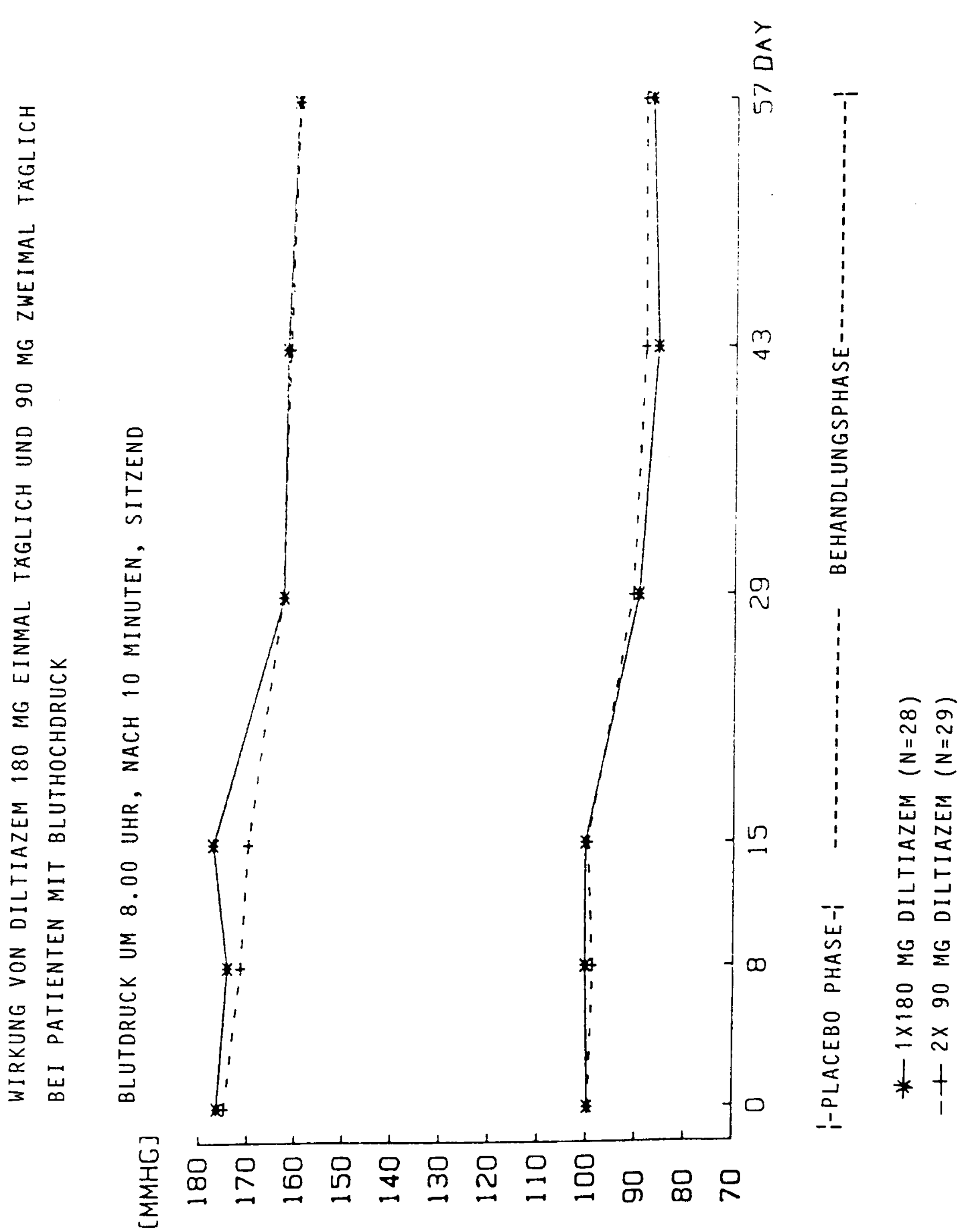
WIRKUNG VON DILTIAZEM 180 MG EINMAL TÄGLICH UND 90 MG ZWEIMAL TÄGLICH
BEI PATIENTEN MIT BLUTHOCHDRUCK
BLUTDRUCK UM 8.00 UHR, NACH 10 MINUTEN, SITZEND
[MMHG]
180
170
160
150
140
130
120
110
100
90
80
70
0
8
15
29
43
57 DAY
!-PLACEBO PHASE-!
BEHANDLUNGSPHASE
1X180 MG DILTIAZEM (N=28)
2X 90 MG DILTIAZEM (N=29)

ABB.6

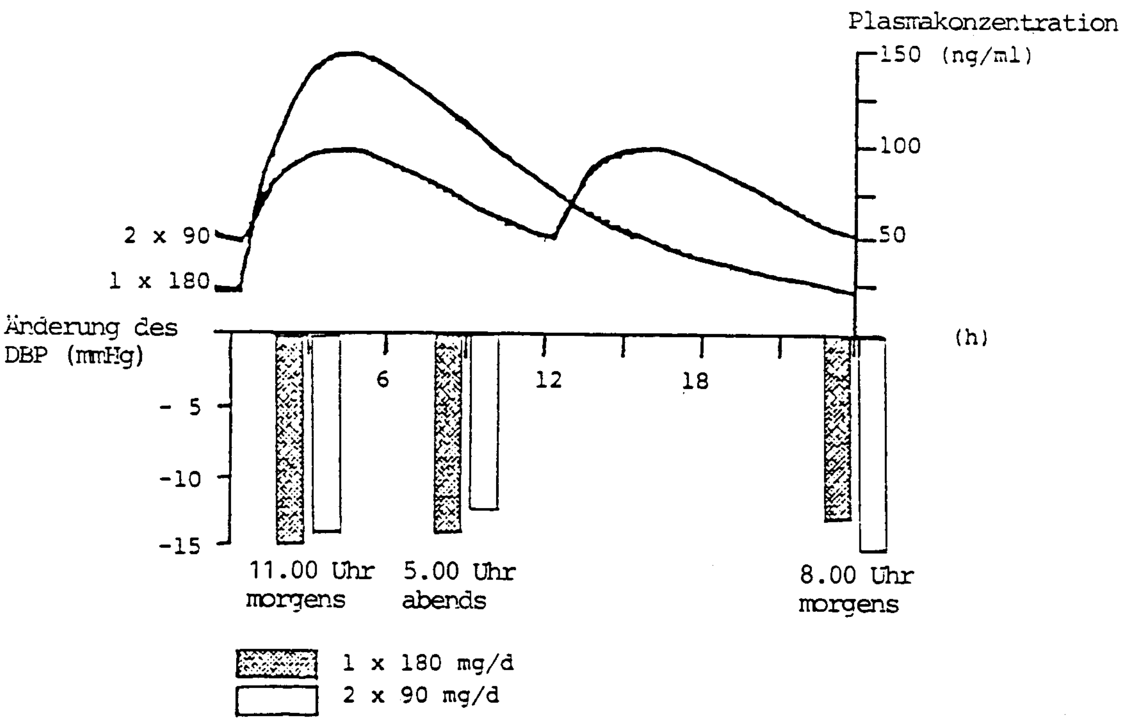


DBP = Diastolischer Blutdruck

ABB.7

WIRKSAMKEIT VON DILTIZEM 180 MG EINMAL TÄGLICH UND 90 MG ZWEIMAL TÄGLICH
IN PATIENTEN MIT BLUTHOCHDRUCK

DIASTOLISCHER BLUTDRUCK NACH 10 MINUTEN SITZEN,
MITTELWERTE ZWEIER AUFEINANDERFOLGENDER MESSUNGEN